## Europäisches Patentamt

## European Patent Office

## Office européen des brevets

(19)

(11) Veröffentlichungsnummer: **0 161 404**
**B1**

(12) **EUROPÄISCHE PATENTSCHRIFT**

(45) Veröffentlichungstag der Patentschrift: 26.09.90

(21) Anmeldenummer: 85102586.6

(22) Anmeldetag: 07.03.85

(51) Int. Cl.⁵: **G 01 N 33/532,** C 07 K 3/00, C 07 K 15/00

(54) Verfahren zur Herstellung von künstlichen biomimetischen Haptenen bzw. Antigenen.

(30) Priorität: 09.05.84 DE 3417022
16.05.84 EP 84105554
16.10.84 DE 3437757
15.11.84 DE 3441764
19.01.85 DE 3501705

(43) Veröffentlichungstag der Anmeldung:
21.11.85 Patentblatt 85/47

(45) Bekanntmachung des Hinweises auf die Patenterteilung:
26.09.90 Patentblatt 90/39

(84) Benannte Vertragsstaaten:
AT BE CH FR GB IT LI LU NL SE

(56) Entgegenhaltungen:
EP-A-0 119 629
EP-A-0 133 540
EP-A-0 139 389
EP-A-0 142 345

(73) Patentinhaber: Theurer, Karl Eugen, Prof.Dr.med.
Brunnwiesenstrasse 23
D-7302 Ostfildern 1 (DE)

(72) Erfinder: Theurer, Karl Eugen, Prof.Dr.med.
Brunnwiesenstrasse 23
D-7302 Ostfildern 1 (DE)

(56) References cited:

NATURE, Band 303, Nr. 5918, Juni 1983, Seiten 627-629, Macmillan Journal Ltd, London, GB; J.W. FORSTROM et al.: "Immunization to a syngeneic sarcoma by a monoclonal auto-anti-idiotypic antibody"

NEW ENGLAND JOURNAL OF MEDICINE, Band 306, Nr. 9, 1982, Seiten 517-522, Boston, US; R.A. MILLER et al.: "Treatment of B-cell lymphoma with monoclonal anti-idiotype antibody"

SURVEYS OF IMMUNOLOGICAL RESEARCH, Band 2, 1983, Seiten 302-305, Basel, CH; J.C. VENTER: "Monoclonal and anti-idiotypic antibodies and the elucidation of receptor structure"

Courier Press, Leamington Spa, England.

**Beschreibung**

Die Immunprophylaxe und -therapie durch aktive und passive Immunisierung ist ebenso wie die Immundiagnostik weiterhin von größter praktischer Bedeutung. Es werden dazu die jeweiligen natürlichen Antigene bzw. Haptene gebraucht. Diese müssen zuerst aus einem Stoffgemisch isoliert werden und stehen oft nicht in ausreichender Menge und Qualität zur Verfügung. Nach Verbrennungen und beim akuten Strahlensyndrom treten Toxine auf, die sekundär zur zusätzlichen Schädigung des Individuums führen (vgl. K. Theurer: Immunologische Prophylaxe und Therapie des akuten Strahlensyndroms: Atompraxis H. 9, S. 327—330, 1958). Auch die Gewinnung von tumorassoziierten Antigenen (Tumormarkern) oder anderen Zellfaktoren, die vom normalen abweichen oder gegen die sich im Organismus eine Autosensibilisierung ausgebildet hat, stößt auf Schwierigkeiten. Für diagnostische wie therapeutische Zwecke sind größere Antigenmengen erforderlich. Die Antigene sollten dabei möglichst rein bzw. isoliert zur Verfügung stehen.

Vorliegendes Verfahren ermöglicht die fortlaufende Gewinnung von biomimetischen imitierenden Stoffen, analog den natürlichen Haptenen, Antigenen oder Toxinen. Die natürlichen Stoffe werden aus Individuen gewonnen und isoliert, die vorher der Noxe ausgesetzt waren oder abweichende Zellfaktoren enthalten. Haptene werden zu Voll-Antigenen komplettiert und diese wie Antigene zur Gewinnung eines primären Antikörpers nach konventionellen Methoden in einem gesunden Individuum in vivo oder in vitro aus Kulturen von Hybridomzellen verwendet. Das Antikörpergemisch kann an Normalgewebe durch Absorptionschromatographie eingeengt und aus den Antikörpern die antideterminanten Gruppen abgetrennt werden. Dieses Verfahren bedeutet eine spezielle Anwendung des Verfahrens zur Synthese von biologischen Peptidwirkstoffen (DE—A—2819110 vom 29.4.1978). Die antideterminanten Fragmente der ersten Antikörper oder auch die gesamten Antikörper werden zur Erzeugung eines zweiten Antikörpers ebenfalls in einem gesunden Indiduum oder in vitro verwendet. Dabei entstehen u.a. Anti-Idiotyp-Antikörper mit der gleichen anitgenen Spezifität wie das ursprüngliche Antigen. Da es sich bei Toxinen, die nach Bestrahlungen oder Verbrennungen auftreten, um ein Gemisch von schädlichen Substanzen handelt, ist hier eine Klonierung, d.h. Einengung auf einen Faktor der verschiedenen Anti-Idiotyp-Antikörper nicht erforderlich. Es ist möglich, hier das gesamte Antikörperspektrum zur Gewinnung der Impfstoffe zu verwenden. Für diagnostische Zwecke können die Anti-Idiotyp-Antikörper bzw. die daraus gewonnenen Faktoren einzeln oder als Konjugate mit Tracerstoffen (Radio-Nucliden), Enzymen oder Farbstoffen markiert werden, wie dies für die Markierung von Antigenen bzw. von Antikörpern bekannt ist (vgl. Lehrbücher der Immunbiologie).

Bezüglich der Idiotypen der natürlichen Antigene und Haptene gibt es jeweils ein Spektrum verschiedener Anti-Idiotyp-Antikörper. Durch Kgonierung der in vitro aus Hybridomzellen gewonnenen Anti-Idiotyp-Antikörper lassen sich aber auch weitgehend isolierte biomimetische Antigene bzw. Haptene gewinnen. Die Gewinnung von Antikörpern sollte möglichst in nicht vorsensibilisierten Individuen und Zellen erfolgen. Für die Erzeugung des primären Antikörpers wie auch für die Anti-Idiotyp-Antikörper können wie in DE—A—2819110 Adjuvantien und Verfahren zur Komplettierung durch Konjugation mit einem Carrier Verwendung finden.

Das vorliegende Verfahren führt zur Verbesserung der biomimetischen Effektivität der Anti-Idiotyp-Antikörper bzw. der daraus gewonnenen Fragmente dadurch, daß mindestens zwei Faktoren der verschiedenen Einzelkomponenten, die ein natives Antigen oder Hapten imitierten, nach bekannten Verfahren der Konjugation von Haptenen bzw. Antigenen chemisch kovalent miteinander verbunden werden. Die Wirksamkeit wird noch weiter verstärkt durch die zusätzliche Einbeziehung von Trägerstoffen in das Konjugat. Diese können Proteine, Nukleinsäuren und Kohlehydrate oder Lipoide, wie auch deren Bestandteile sein (vgl. DE—A—3417022 Träger- und Begleitstoffe für biologische Wirk- und Regulationsfaktoren aus Zellen und Geweben und deren Anwendung).

Vorliegendes Verfahren kann also allgemein zur Gewinnung von biologischen bzw. therapeutischen biomimetischen Wirkstoffen, welche die Wirkung von natürlichen Stoffen imitieren, verwendet werden. Es lassen sich alle geeigneten und bekannten Verfahren der chemischen Konjugation von Peptiden und Proteinen, wie auch die Konjugation derselben mit Nukleinsäuren, Kohlenhydraten und Lipiden sowie deren Untereinheiten bzw. Bestandteile verwenden.

Dabei sollte die Stereospezifität des Konjugats dem Idiotyp-Molekül entsprechen, sodaß eine Bindung mit dem jeweiligen natürlichen Rezeptor-Molekül möglich ist. Das Verfahren eignet sich deshalb für die Partialsynthese von Molekülen mit bestimmten biologischen Wirkungen. Die biologisch aktiven Bezirke, insbesondere der Anti-Idiotyp-Antikörper und deren Fragmente müssen deshalb nach außen gerichtet sein.

Eine derartige Kopplung, ggfs. zusätzlich an biologische Trägerstoffe, ebenso wie die Verwendung von Radio- und Verbrennungstoxinen als Ausgangsstoffe, ist neu nach dem Stand der Technik.

In Nature, Band 303, Nr. 5918 vom Juni 1983, S. 627—629, London werden Anti-Idiotyp-Antikörper allgemein als imitierende Antigene mit spezifischer Immunantwort bezeichnet. Im vorliegenden Falle handelt es sich jedoch um besondere Ausgangsstoffe in Form von Toxinen, die nach Bestrahlung mit ionisierenden Strahlen oder Verbrennungen auftreten, die nur nach entsprechender Vorbehandlung eines Spenders und nicht unmittelbar auftreten, ebenso um die Konjugation von Anti-Idiotyp-Antikörpern.

In der Literaturstelle "New England Journal of Medicine" Bd. 306, Nr. 9 S. 517—522, Boston von 1982 handelt es sich um die Behandlung von Blutkrebs mit monoklonalen Antikörpern, die nicht konjugiert werden.

In "Surveys of Immunological Research" Bd. 2, S. 302—305, Basel von 1983 werden ebenfalls monoklonale Anti-Idiotyp-Antikörper zur Imitation von Insulin verwendet.

In EP—A—0 119 629 wird allgemein die funktionelle Wirkung als Antigene und Haptene der Anti-Idiotyp-Antikörper diskutiert und keine Konjugation derselben angegeben. Hier ist die Markierung der Anti-Idiotyp-Antikörper und deren Fragmente beschrieben.

In EP—A—0 133 540 von 1984 wird über die Art der Bindung der idiotypischen Determinanten und der Hemmung dieser Bindung auf Antiidiotypische Antikörper diskutiert. Auch dies bezieht sich nicht auf die chemisch kovalente Bindung von mindestens zwei Bestandteilen des Spektrums. Es werden hier lediglich mehrere Moleküle des Markierungsstoffes mit einem monovalenten Anti-Idiotyp-Antikörper konjugiert.

In EP—A—0 139 389 von 1984 wird die Gewinnung von anti-idiotypischen monoklonalen Antikörpern in Hybridomzellen beschrieben sowie die Markierung derselben für den Immunassay, nicht jedoch die chemisch kovalente Konjugation verschiedener Faktoren bezüglich der Epitope des Antigens. Die monoklonalen Anti-Idiotyp-Antikörper werden mit dem ursprünglichen Antigen mitverwendet.

In EP—A—0 142 345 von 1984 wird ein kombiniertes Antiidiotyp-Vakzin durch Mischung, jedoch nicht durch chemisch kovalente Bindung von verschiedenen Anti-Idiotyp-Antikörpern gewonnen. Der Anti-Idiotyp-Antikörper oder sein Fragment wird hier lediglich mit einem Adjuvans verbunden, nicht aber verschiedene Anti-Idiotyp-Antikörper durch chemische Bindung zu einem neuen Molekül vereinigt.

Die chemische Konjugation der Anti-Idiotyp-Antikörperfragmente kann nach verschiedenen, bereits bekannten Verfahren erfolgen analog der Herstellung von künstlich zusammengesetzten Antigenen, Proteinen und Proteinen, so z.B. durch Kupplung als Diazonium-Verbindung, die Behandlung mit Zyanaten, die Umsetzung mit Carbobenzoxy-Verbindungen, das Curtius'sche Azid-Verfahren, das Oxazolon-Verfahren, die Konjugation mit Polysacchariden nach W. J. T. Morgan, Verbindung vit Carboxyhydraten, Pyridin-Eiweiß-Verbindungen, Verbindungen mit Sulfhydril-Gruppen zu Disulfid-Brücken u.a. (vgl. A. Schmidt: Fortschritte der Serologie, S. 67 u.f., Dietrich Steinkopf Verlag, Darmstadt, 1955; Helmut Friemel: Immunologische Arbeitsmethoden, S. 480 u.f., Gustav Fischer Verlag, 1984).

Der Nachweis der erfolgten Konjugation der Ausgangsstoffe wird durch bekannte physikalische oder immunbiologische Vergleichsuntersuchungen geführt, wie z.B. die Bestimmung der Molekulargewichte, chromatographische und elektrophoretische Auftrennungen, insbesondere durch Hochdruck-Flüssigkeitschromatographie, Immunelektrophorese u.a. Der Nachweis der Wirkung erfolgt durch Bioassay in vitro an Zell- und Gewebekulturen, an zellfreien Synthesesystemen oder pharmakologisch im Tierversuch.

Die Anwendung der Konjugate erfolgt für diagnostische wie auch therapeutische Zweke in Human- und Veterinärmedizin, wie auch zur Beeinflussung von Zellkulturen, Mikroorganismen und Pflanzen.

Beispiel 1

Synthese von künstlichen biomimetischen Antigenen analog den tumorassoziierten Antigenen aus Tumorgewebe. Sie erfolgt in gleicher Weise wie in DE—A—2 819 110 Beispiel 1. Es werden nach bekannten Aufbereitungsverfahren die jeweiligen Tumormarker isoliert und in konventioneller Weise zur Sensibilisierung von Antikörper-erzeugenden Tieren bzw. zur Sensibilisierung von Hybridomzellen verwendet. Es können auch embryo-fetale Zellmarker, die bei Tumorzellen wieder auftreten, in gleicher Weise verarbeitet werden.

Die entstehenden primären Antikörper werden durch Bioassay auf ihre Fähigkeit zur Blockierung der biologischen Wirkung des urprünglichen Antigens bzw. Haptens, das zum Voll-Antigen komplettiert wurde, ggfs. unter Verwendung von radiologischen Tracermethoden getestet. Durch Absorption und Absättigung an Normalgewebe wird der spezifische Antikörper oder das Antikörperspektrum gereinigt und zum Antigen komplettiert, oder aber die antideterminanten Gruppen als Antikörperfragmente isoliert und ebenfalls zum Antigen komplettiert, bevor man sie zur Gewinnung von Anti-Idiotyp-Antikörpern in einem anderen, nicht vorsensibilisierten Immunsystem verwendet. Auch hier kann die Antikörpersynthese in vitro über Hybridomzellen erfolgen. Die gewonnen Anti-Idiotyp-Antikörper können wie die daraus gewonnen antideterminanten Fragmente zum diagnostischen Nachweis entsprechender primärer Antikörper aus den Körperflüssigkeiten durch Antigen-Antikörperreaktionen verwendet werden. Der primäre Antikörper und die aus ihm gewonnenen antideterminanten Gruppen lassen sich therapeutisch wie auch diagnostisch entsprechend DE—A—2 456 224 verwenden. Dabei werden diese mit Zytostatika oder zytotoxisch wirksamen Substanzen oder mit Radionukliden gekoppelt.

Beispiel 2

Gewinnung von immunogenen, die Wirkung von toxischen Faktoren, wie sie bei Letalbestrahlung mit ionisierenden Strahlen in einem Individuum entstehen, biomimetisch imitierenden Anti-Idiotyp-Antikörpern.

Die Gewinnung der "Radiotoxine" erfolgt wie in Atompraxis H. 9, S. 327—330, 1958, angegeben. Außer Blutlysat kann dazu auch Gewebehomogenat, insbesondere aus Knochenmark, Leber und Milz verwendet werden. Wie in Beispiel 1 wird die Immunogenität der Faktoren zur Gewinnung von primären Antikörpern in einem nicht vorsensibilisierten Immunsystem in vivo oder in vitro verstärkt. Die daraus gewonnenen Antikörper werden gegebenenfalls an Normalgewebe absorptionschromatographisch gereinigt und wie in Beispiel 1 zu Anti-Idiotyp-Antikörpern weiterverarbeitet. Diese werden zum Antigen komplettiert und zur Prophylaxe bzw. Therapie von Strahlenschäden als Impfvaccine oder zu Diagnostika verwendet.

Beispiel 3

Gewinnung von "Verbrennungstoxinen"—ähnlichen Antigenen bzw. Haptenen. Ausgang dafür sind verbrennungsgeschädigte Individuen aus deren Blut oder Körpergewebe die Verbrennungstoxine isoliert werden. Nach DE—A—3441764.8 können diese ebenso wie die Strahlentoxine auch aus entsprechend geschädigten Gewebe- und Zellkulturen gewonnen werden. Die weitere Verarbeitung zu Anti-Idiotyp-Antikörpern bzw. -fragmenten erfolgt wie in Beispiel 1 und 2 angegeben. Es können Kombinationsimpfstoffe aus biomimetisch wirkenden Strahlentoxinen und Verbrennungstoxinen durch Kombination beider Faktoren gewonnen werden.

Beispiel 4

Nach DE—A—2 819 110 und DE—A—3 437 754.3 werden nach konventionellen Methoden oder aus in vitro-Zellkulturen von Lymphozyten oder Hybridom-Zellen Anti-Idiotyp-Antikörper gewonnen (vgl.: Herwart Ambrosius: Antiserumgewinnung aus Tieren, S. 25 und S. Wichner: Antikörperbildung in der Zellkultur, S. 48—in H. Friemel: Immunologische Arbeitsmethoden: Gustav Fischer Verlag, Stuttgart, 1984). Von den Anti-Idiotyp-Antikörpern werden die antideterminanten Fragmente enzymatisch abgespalten und chromatographisch isoliert. Bei Verwendung eines Proteins oder Proteids als Ausgangsstoff (Idiotyp-Molekül) z.B. einem bestimmten Enzym wie der GPT (Glutamat-Pyruvat-Transaminase) entstehen mehr als zehn verschiedene primäre Idiotyp-Antikörper gegenüber den verschiedenen Oberflächenbezirken des Moleküls. Von diesen Antikörpern werden die N-terminalen Bezirke bzw. antideterminanten Gruppen als Antigen für die Erzeugung von entsprechenden Anti-Idiotyp-Antikörpern verwendet. Die Gewinnung derselben erfolgt möglichst in einem autologen System bezüglich der primären Antikörper, weil dort der Toleranzbruch gegenüber den variablen Bezirken des primären Antikörpers und die Entstehung von Anti-Antikörpern mit Idiotyp-Eigenschaften gegenüber einem Toleranzbruch bezüglich der konstanten Bezirke, der nicht die funktionellen Gruppen betreffen würde, erleichtert ist. Von den isolierten antideterminanten, N-terminalen Bezirken der Anti-Idiotyp-Antikörper werden nun mindestens zwei Moleküle miteinander konjugiert. Wenn die Fragmentierung z.B. für die leichten und schweren Ketten der Antikörper durch Aufspaltung mit Mercaptoäthanol an den Disulfid-brücken erfolgt und eine Rekombination bzw. Reaggregation durch bekannte Methoden verhindert wird, können die isolierten Fragmente durch Bindung der freien SH-Gruppfen konjugiert werden. Ohne Bindeglied lassen sich auch Konjugate aus Polysacchariden gewinnen, die mit einer begrenzten Anzahl von Fettsäureresten substituiert sind. Diese adsorbieren sich direkt an den Proteinen bzw. Peptiden (Hämmerling, U.; O. Westphal: Europ. J. Biochem. 1, 46 (1967)). Besonders geeignet zur Konjugation sind auch die $Fab_1$- und $Fab_2$-Fragmente der Anti-Idiotyp-Antikörper.

Bei den Antikörperfragmenten kann auch die Tannin-Methode angewandt werden (Borduas, A., P. Grabar: Ann. Inst. Pasteur 84, 903 (1953)); Stravitsky, A.: J. Immunol, 72, 306 (1954); Roitt, I. und Doniach, D.: WHO-Book of immunologic techniques, S. 20: Wold Health Organisation, Genf (1966).

Auch die Benzidin-Methode ist dazu geeignet (Gordon, J.; B. Rose, A. Sekon: J. exp. Med. 108, 37 (1958)); Roberts, I., D. Doniach: WHO-Book of immunologic techiques, S. 1: World Health Organisation, Genf (1966); Stravitsky A., E. Arquilla: J. Immunol. 74, 306 (1955); Timpe, R., H. Furthmayr, I. Wolff: Int. Arch. Allergy 32, 318 (1967).

Von J. Brock wurde die Konjugation durch Nitrobenzolsulfonat publiziert (vgl. Präparation von konjugierten Antigenen, S. 481 in H. Friemel: Immunologische Arbeitsmethoden: Gustav Fischer Verlag, Stuttgart (1984). Nach Beendigung der Reaktion wird das DNP-Produkt durch Gel-Chromatographie an Sephadex gereinigt.

Beispiel 5

Es werden nach den benannten Methoden Konjugate aus antideterminanten Bezirken von Anti-Idiotyp-Antikörpern bezüglich dem somatotropen Hormon (Wachstumshormon) gewonnen.

Beispiel 6

Es werden Konjugate aus Anti-Idiotyp-Antikörperfragmenten mit der Wirkung des Enzymes Arginase hergestellt. Dazu werden als urspezifische Trägersubstanz das Kohlenhydrat Dextran mit einem Mol. gewicht kleiner als 10.000 verwendet.

In gleicher Weise können auch Sephadex-Ionenaustauscher für die Konjugation verwendet werden.

Beispiel 7

Zur Konjugation kommen Anti-Idiotyp-Antikörperfragmente mit Nuckleoditen oder Desoxinukleotiden, ggfs. in Kombination mit Kohlenhydraten und/oder Lipiden.

Beispiel 8

Zur Konjugation kommen Anti-Idiotyp-Antikörperfragmente mit Lipiden und Peptiden als Fragmente von Organextrakten mit einem Mol.gewicht von 3000 Dalton, die nach DE—A—3 417 022.7-44 gewonnen wurden.

Beispiel 9

Es werden mehrere Anti-Idiotyp-Antikörper bezüglich eines Idiotyp-Moleküls oder daraus gewonnene Fragmente mit antideterminanten Bezirken oder Konjugate von diesen, ggfs. unter Einbeziehung eines Trägerstoffes mit Tracermolekülen für den immunologischen Nachweis einer humoralen oder zellulären Sensibilisierung konjugiert (vgl.: Markierung von Proteinen: Hans Arthur Schulze in H. Friemel: Immunologische Arbeitsmethoden: Gustav Fischer Verlag, Stuttgart, (1984); Rodbard, D., Weiß, G. H.: Mathematical theory of immunoradiometric (labeled antibody) assay; Anal. Biochem. 52, 10 (1973).

**Patentansprüche**

1. Verfahren zur Herstellung von künstlichen biomimetischen Haptenen bzw. Antigenen analog der Herstellung biologischer Peptidwirkstoffe unter Verwendung eines isolierten Peptidwirkstoffes zur Gewinnung primärer Antikörper, bei welchen man gegen die antideterminante Gruppe oder Fragmente des primären Idiotyp-Antikörpers Anti-Idiotyp-Antikörper bzw. deren determinante reaktive Gruppen in Form leichter oder schwerer Ketten oder deren Fab-Fragmente erzeugt und diese ggfs. durch Tracerstoffe markiert und Mischungen bezüglich verschiedener Ausgangsstoffe kombiniert dadurch gekennzeichnet, daß aus dem jeweiligen Gesamtspektrum verschiedener Anti-Idiotyp-Antikörper bzw. -Fragmente, die gegen verschiedene Idiotypen bezüglich eines Haptens oder Antigens gerichtet sind, mindestens zwei Bestandteile gegebenenfalls mit biologischen Trägerstoffen chemisch kovalent konjugiert werden.

2. Verfahren nach Anspruch 1, dadurch gekennzeichnet, daß Anti-Idiotyp-Antikörper bzw. -Fragmente verwendet werden, die gegen Idiotypen bezüglich Toxinen gerichtet sind, welche nach Schädigung von Individuen mit ionisierenden Strahlen und/oder Verbrennungen entstehen.

**Revendications**

1. Procédé destiné à la fabrication d'haptènes ou d'antigènes biologiques synthétiques, analogue à la fabrication de principes actifs peptidiques biologiques, avec utilisation d'un principe actif peptidique isolé pour l'obtention d'anticorps primaires pour lesquels on engendre contre le groupe antidéterminant ou des fragments de l'anticorps idiotype primaire des anticorps anti-idiotypes ou leurs groupes réactifs déterminants, sous forme de chaînes simples ou complexes, ou leurs fragments Fab, on les marque le cas échéant avec des traceurs et on combine des mélanges à partir de difféentes substances initiales caractérisé par le fait que parmi la gamme complète des différents anticorps ou fragments anti-idiotypes qui s'opposent à différents idiotypes en ce qui concerne un haptène ou un antigène, au moins deux, composants sont, le cas échéant, conjugués chimiquement par covalence avec des substances porteuses biologiques.

2. Procédé selon la prétention 1, caractérisé par le fait qu'on utilise des anticorps ou des fragments anti-idiotypes qui s'opposent aux idiotypes en ce qui concerne les toxines qui se forment après lésion d'individus par des rayons ionisants et/ou des brûlures.

**Claims**

1. Procedure for the manufacture of synthetic biomimetic haptens/antigens in analogous fashion to the production of biological peptide principles using an isolated peptide principle for the production of primary antibodies in which anti-idiotype antibodies to the antideterminant group or fragments of the primary idiotype antibody or their determinant reactive groups are produced in the form of light or heavy chains or their fab fragments and (as appropriate) labeled by suitable tracers and mixtures relative to various starting materials are combined, whose distinctive feature is that at least two components of the respective total spectrum of different anti-idiotype antibodies or fragments directed againt various idiotypes relative to a hapten or antigen are, as appropriate, conjugated with biological carriers by covalent bonds.

2. Procedure from Claim 1 which procedure is characterized by the use of anti-idiotype antibodies or fragments directed against idiotypes relative to toxins produced in individuals sustaining damage following exposure to ionizing radiation and/or burns.